# EUROPEAN PATENT SPECIFICATION

(11) **EP 4 213 816 B1**
(45) Date of publication and mention of the grant of the patent: **10.07.2024**
(21) Application number: 21766528.0
(22) Date of filing: 08.09.2021
(51) Int. Cl.: A61K 9/50, A61K 9/51, A61K 49/00

(54) **MULTI-TARGETING CELL-DERIVED NANOPARTICLES AS A VERSATILE THERANOSTIC**
AUF MEHRERE ZIELE GERICHTETE NANOPARTIKEL ALS VIELSEITIGE THERANOSTISCHE THERAPIE
NANOPARTICULES DÉRIVÉES DE CELLULES À CIBLAGE MULTIPLE EN GUISE DE PRODUIT THÉRANOSTIQUE POLYVALENT

(30) Priority: 16.09.2020 IT 202000021871
(43) Date of publication of application: 26.07.2023
(73) Proprietor: Fondazione Istituto Italiano di Tecnologia, 16163 Genova (GE) (IT)
(72) Inventor: TAPEINOS, Christos, 56025 Pontedera PI (IT); CIOFANI, Gianni, 56025 Pontedera PI (IT)
(74) Representative: Perani & Partners S.p.A.
(86) International application number: PCT/IB2021/058163
(87) International publication number: WO 2022/058843

(56) References cited:
- US-A1- 2018 036 240
- CHEN HONG-YING ET AL: "Hybrid cell membrane-coated nanoparticles: A multifunctional biomimetic platform for cancer diagnosis and therapy", ACTA BIOMATERIALIA, ELSEVIER, AMSTERDAM , NL, vol. 112, 26 May 2020 (2020-05-26), pages 1 - 13, XP086212263, ISSN: 1742-7061, [retrieved on 20200526], DOI: 10.1016/J.ACTBIO.2020.05.028
- DIANA DEHAINI ET AL: "Erythrocyte-Platelet Hybrid Membrane Coating for Enhanced Nanoparticle Functionalization", ADVANCED MATERIALS, vol. 29, no. 16, 15 February 2017 (2017-02-15), pages 1 - 8, XP055573232, ISSN: 0935-9648, DOI: 10.1002/adma.201606209

## Description

### FIELD OF THE INVENTION

The present invention concerns drug delivery systems, specifically cell-membrane-nanoparticles, for controlling the delivery and the release of the administered drug.

### STATE OF THE ART

There is a high number of therapeutic, diagnostic and theranostic substances/molecules/structures that can be used for the treatment of life-threatening diseases (e.g., ischemic stroke, myocardial infarction, atherosclerosis and cancer) [1-4]. However, these substances/molecules/structures cannot safely and efficiently be delivered into the diseased tissue due to several reasons. Some of these reasons include: 1) the low colloidal stability that may result to undesirable aggregates during blood circulation and subsequent blood clots [5], 2) the fast clearance from the reticuloendothelial system (RES) due to the lack of a counter opsonization ability or due to the aforementioned aggregation [6], and finally 3) the non-controlled biodistribution due to the lack of tissue specificity [7]. In certain cases, other physical and anatomical barriers like the blood-brain barrier or the pancreatic desmoplastic stroma, inhibit further the safe delivery of these theranostics, resulting in reduced therapeutic and/or diagnostic efficiency. To overcome these limitations, these substances/molecules/structures can be encapsulated inside properly designed matrices, named drug delivery systems (DDS).

When a drug is administered to an individual, a DDS that controls the delivery and release of the administered drug into cells, tissues, and organs is very important for optimal efficacy. When the DDS is applied, advantages such as maximizing the efficacy and stability of a drug, prolonging its residence time, increasing its bioavailability, and minimizing its side effects can be achieved. Thus, the DDS can be considered as important as the therapeutic component itself. Since the early 1960s, DDSs have been developed in various forms.

Artificial systems, such as liposomes, have been widely studied as drug delivery systems. Liposomal preparations have low toxicity and are highly applicable because the size, charge, membrane composition, and membrane permeability of the liposomal preparations may be controlled according to prescription. Recently, stealth liposomes prepared by conjugating liposomes with a polymer, such as polyethylene glycol (PEG), have been developed. The stealth liposomes increase the circulatory half-life of drugs by preventing drug-containing liposomes from being easily removed from the blood. However, since liposomes and stealth liposomes lack the ability to recognize specific types of cells, they may not be used for the purpose of delivering drugs to specific types of cells or tissues, unless they are conjugated with antibodies specific to a target cell.

The use of DDS provides improved colloidal stability, stealth ability against RES clearance, advanced targeting specificity, and enhanced internalization by specific tissues. Although these delivery systems result in an overall boosted therapeutic efficiency, they deem insufficient in real conditions. The reasons for this inadequacy are several and include undesirable toxicity due to their biodegradation products or their non-controlled biodistribution to healthy tissues, low encapsulation efficiency that results in higher doses that cannot be safely tolerated from patients, and RES clearance since their stealth ability is lost when they administered into patients. Furthermore, in the case of the physical/anatomical barriers, even if the crossing ability of these delivery systems is improved, the high doses needed to enter the diseased tissue and demonstrate a therapeutic effect cannot be achieved.

Recently, a further improvement to these systems was achieved by combining organic and/or inorganic cores with a biomimetic shell derived from the membrane of various cells. [8, 9] This strategy led to remarkable improvements in stability, cells' internalization, and tissue specificity, allowing for an enhanced internalization and improved tissue specificity [10-14].

Another example of drug delivery system comprising an organic and/or an inorganic core enclosed in a biomimetic shell is provided by D. Dehaini and colleagues (D. Dehaini et al., "Erythrocyte-Platelet Hybrid Membrane Coating for Enhanced Nanoparticle Functionalization", 2017 HHS Public Access), that prepared dual-membrane-coated nanoparticles obtained by fusing RBC (Red Blood Cells) membrane and platelet membrane. Membrane-coated nanoparticles were fabricated by preparing poly(lactic-co-glycolic acid) (PLGA) cores, and then coating them with either RBC membrane, platelet membrane, or fused RBC-platelet membrane. A mixture of PLGA cores and membrane material was then sonicated using a Fisher Scientific FS30D bath sonicator for 2 minutes to form the final coated nanoparticles.

In US2018/0036240 A1 cell membrane-derived nanovesicles are disclosed; their production method provides for the extraction of cell membranes from cells, the intracellular components of which have been removed by treating the cells with an alkaline solution of pH 9 to 14, and a size of the nanovesicles is smaller than a size of the cells. The document discloses also a method of preparing cell membrane-derived nanovesicles loaded with substances for treating or diagnosing diseases, the method comprising: (a) a step of removing intracellular materials by treating cells with an alkaline solution of pH 9 to 14; (b) a step of adding therapeutic or diagnostic substances into a suspension containing membranes of the cells from which the intracellular materials have been removed; (c) a step of preparing nanovesicles by applying a sonication method to the suspension to which the therapeutic or diagnostic substances are added; and (d) a step of isolating the prepared nanovesicles from the suspension. It is worth noting that the method disclosed therein is suitable for the production of nanoparticles comprising cell membranes derived from a single cell line (see examples 1-13 wherein monocytes-derived or transformed cells-derived nanoparticles are characterised).

Chen Hong-Ying et al, in the publication entitled "Hybrid cell membrane-coated nanoparticles: A multifunctional biomimetic platform for cancer diagnosis and therapy" (Acta Biomaterialia 112 (2020), 1-13), review the preparation strategies, camouflaging mechanism and the antitumor applications of hybrid cell membrane-camouflaged nanoparticles (NPs). The authors report that hybrid cell membrane-derived vescicles can be successfully prepared by the extrusion method after cell membrane isolation and extraction. Sonication can also be used, before extrusion, to fabricate the hybrid cell membrane-derived vescicles, to expedites their preparation method.

Studies on nanovesicles directly derived from the membranes of cells prove that removing the intracellular components, which are unnecessary for targeting, can increase the efficiency of drug loading and prevent side effects when administered into the body. Thus, the development of systems that are lacking their intraluminal components is considered very promising.

Since cell-derived delivery systems use the natural targeting systems of the cells, targeting may be easily induced. Studies on drug delivery systems that use extracellular vesicles, which are naturally secreted from cells, or nanovesicles, artificially prepared from nucleated mammalian cells, have been reported.

The increasing demand in injectable DDS using technologically advanced and smart nanotherapeutics is attributed to patients' needs suffering either from chronic diseases (e.g., diabetes, Parkinson's disease, chronic obstructive pulmonary disease), as well as life-threatening diseases including cancer, ischemic stroke, and myocardial infarction. Although the evolution of nanomedicine resulted in the fabrication of specific delivery systems with improved localization in hard-to-target diseased tissues, as well as controlled release of their therapeutics/diagnostics, still there is an immense need for the fabrication of a versatile nanoplatform that can be used as a multi-theranostic approach targeting a variety of diseases.

### Technical Problem of the prior art

Although the biomimetic shell improves the internalization and the tissue specificity of the DDS, the undesirable toxicity due to the biodegradation products of the organic and/or inorganic core remains unsolved.

Cell membrane nanovesicles composed by cells of a single type only have the ability to target with high precision cells of the same type.

Recent studies have reported that it is possible to use different types of cell membrane to form hybrid nanoparticles, since these hybrid nanoparticles carry properties from both the cell types.

Nonetheless, the majority of the current methods for collecting the extracted cell membranes demands ultra-centrifugation. The use of ultracentrifugation increases the cost significantly due to the high price of both the equipment as well as the consumables.

Moreover, the alkaline pH used in the prior art for the fabrication of artificial nanovesicles denatures the proteins of the membrane, reducing their targeting ability. In addition, this denature will lead to conformational changes of various transmembrane proteins reducing further the stability of a fabricated vesicle. Besides, the alkaline pH may act as a limiting factor for the combination of more than one cell membrane in one vesicle type, since the high ionic strength and the enhanced surface charge will counteract potential hydrophobic interactions that keep the different membranes together.

### SUMMARY OF THE INVENTION

The main object of the present invention is the provision of a simpler and straightforward way for fabricating hybrid cell membrane derived nanoparticles that combine multi-targeting capacities with high specificity and phagocytosis-evasive abilities.

The object of the present invention is therefore a method for the production of cell-membrane derived nanoparticles, comprising the steps of extracting the cell membranes from cells of two or more different cell lines and fusing them by means of high-pressure homogenisation in presence of a disperse therapeutic or diagnostic ingredient.

Another object of the present invention are cell-membrane derived nanoparticles enclosing an active ingredient; these nanoparticles are obtainable by the method of the invention and can be employed as drug delivery systems for medical or diagnostic use.

### Advantages over prior art

Scalability - The use of high-pressure homogenizer allows the extraction of millions and even billions of nanoparticles, as well as the fusion of the cells and the extraction of mixed cell membranes. This allows the extraction and fabrication of fused cell membrane nanoparticles (F-CMNPs) at an industrial scale.

Simplicity - A high-pressure homogenizer, along with the use of filtering techniques respectively for extraction and cleaning, simplifies the procedure and costs five times less than an ultracentrifuge.

High speed - The extraction and cleaning of the membrane can be performed only in 45 minutes and very low speed (ca. 4000 g), where the ultracentrifuge requires at least six hours and high speed (ca. 100000-200000 g).

Multitargeting - The invention enables the fabrication of F-CMNPs with a multi-targeting ability that can be controlled by the selective choice and percentage of the desired cell membranes.

Biocompatibility - The method allows to produce an entirely biocompatible system, since the invented F-CMNPs are comprised only of cell membranes.

High loading capacity - high capacity of loading the nanoparticles with drugs (ca. 25%, which is four times higher than existing nanosystems).

### BRIEF DESCRIPTION OF THE FIGURES

**Figure 1****.** SEM images of A) CMNPs from a combination of U251-MG and hCMEC/cell membranes (UH-CMNPs) and B) CMNPs from a combination of U251-MG, hCMEC/D3 and T98G cell membranes (UHT-CMNPs).
**Figure 2****.** A) Size and B) surface charge distributions of CMNPs from a combination of U251-MG, hCMEC/D3 and T98G cell membranes (UHT-CMNPs)
**Figure 3****.** SDS-PAGE pf various CMNPs. L1: Marker, L2: H-CMNPs, L3: U-CMNPs, L4: UH-CMNPs, L5: UHT-CMNPs, L6: sphingomyelin nanoparticles functionalized with angiopep-2 (SM-AP2) (positive control)
**Figure 4****.** Assessment of internalization using various cancerous and non-cancerous cell-lines treated with hCMEC/D3-derived particles (H-CMNPs), U251-MG-derived particles (U-CMNPs), a combination of U251-MG and hCMEC/D3 (UH-CMNPs), a combination of U251-MG, hCMEC/D3 and T98G (UHT-CMNPs), and as a positive control, sphingomyelin nanoparticles functionalized with angiop-2 (SM-AP2).
**Figure 5****.** % Cell proliferation of the U-251 MG cell line, after 72 h, using various concentrations of the non-encapsulated and encapsulated (UHT-CMNPs) model drug 3PO.

### DETAILED DESCRIPTION OF THE INVENTION

### Method

For a greater intelligibility of the text, the method of production of the nanoparticles will be described step by step, disclosing, whether necessary, the preferred embodiments of the invention for each step.

As mentioned above, the method according to the invention is dedicated to the production of cell-membrane derived nanoparticles and, for this purpose, comprises the following steps:
I) providing from two to four cell cultures, each cell culture being of a different cell line, and collecting a predetermined number of cells from each cell line;
II) lysing the predetermined number of cells of each cell line to obtain lysates of each cell line;
III) extracting cell membranes from the lysate of each cell line to obtain cell membrane dispersions of each cell line;
IV) mixing the cell membrane dispersion of each cell line in amounts such that the corresponding predetermined number of cells collected from each cell line represents from 0.25 to 4 parts of the total number of cells collected in step (I), and adding an aqueous solution or dispersion containing a therapeutic or diagnostic ingredient to obtain a mixed dispersion;
V) homogenising the mixed dispersion at an external air pressure ranging from 0.552 to 1.151 MPa (80 to 167 psi), with a number of passages ranging from 6 to 14, to form a dispersion containing a plurality of nanoparticles;
VI) isolating the plurality of nanoparticles from the dispersion containing them.

According to a preferred embodiment, the method of the invention is performed maintaining the pH < 7.5 at every step, preferably between 5 < pH < 7.5, preferably between 5 < pH < 6.5, preferably at a pH of 5.5 circa.

### Step I: providing two or more cell cultures, each cell culture being of a different cell line.

For the aim of the invention, by cell membrane is meant the cytoplasmic membrane (or plasma membrane), i.e. the biological membrane that separates the interior of a cell from its outside environment.

By cell line is meant a plurality of cells that have been continually passaged over a long period of time (20 - 80 passages) and have acquired homogenous genotypic and phenotypic characteristics. Cell lines can be finite or immortalized.

Preferably, the two or more cell lines used for the aim of the invention are animal or human cell lines, independently selected from the group consisting of primary cell lines and immortalised cell lines, wherein by primary cells is meant cells isolated directly from human or animal tissue, whilst by immortalized (or continuous) cell line is meant a cell line that has acquired the ability to proliferate indefinitely, either through genetic mutations or artificial modifications.

Preferably, immortalised cell lines are used; suitable immortalised cell lines are selected from the group consisting of
- glioblastoma cell lines, including preferably U-251 MG, U87 MG, T98G;
- neuroblastoma cell lines, including preferably SH-SY5Y and its neuronal-like differentiated form (Effects of all-trans-retinoic acid on human SH-SY5Y neuroblastoma as in vitro model in neurotoxicity research, NeuroToxicology 30 (2009) 127-135).
- endothelial cell lines, including preferably hCMEC/D3,
and combinations thereof.

Examples of suitable combinations for the aim of the invention are:
- U-251 MG + hCMEC/D3
- U-251 MG + hCMEC/D3 + T98G
- hCMEC/D3 + Neural cells - SH-SY5Y differentiated.

The advantage of using different cell lines lies in that, depending on the amount of the various cell membrane extracts used to make the nanoparticles, it is possible to tune the nanoparticles properties and, in particular, their internalization time by different cell types. Indeed, the combination of different cell membranes provides the nanoparticles high specificity towards different cell types and overall better internalization results.

According to a preferred embodiment, the method provides for the combined use of cell membranes derived from four different cell lines, wherein the cell membrane of each cell line is mixed in amounts such that the corresponding number of cells collected from each cell line represents preferably from 0.25 to 4 parts, preferably from 0.5 to 3 parts, preferably from 1 to 2 parts, preferably from 1 to 1.5 parts, of the total number of cells used (n. cells/n. cells).

Preferably, the cell membrane of each cell line is mixed in amounts such that the corresponding number of cells collected from each cell line is in a 1:1:1:1 ratio based on the total number of cell used.

For example: UHTS-CMNPs (CMNPs: Cell Membranes Derived NanoParticles) derive by combining 1 part of cell membranes derived from U-251 MG cells, 1 part of cell membranes derived from hCMEC/D3 cells, 1 part of cell membranes derived from T98G cells and 1 part of cell membranes derived from SH-SY5Y cells.

In other words, the amount of cell membranes combined together is inferred based on (considered equal to) the number of cells used for extracting the corresponding cell membranes (e.g.: a cell membrane mixture comprising equal parts - 1:1 ratio - of cell membranes from cell line A and cell membranes from cell line B, is obtained using the cell membrane dispersion extracted from 3.5×10⁶ cells of cell line A and 3.5×10⁶ cells of cell line B).

According to a preferred embodiment, the method provides for the combined use of cell membranes derived for three different cell lines, wherein the cell membrane of each cell line is mixed in amounts such that the corresponding number of cells collected from each cell line represents preferably from 0.25 to 4 parts, preferably from 0.5 to 3 parts, preferably from 1 to 2 parts, preferably from 1 to 1.5 parts of the total number of cells used.

Preferably, the cell membrane of each cell line is mixed in amounts such that the corresponding number of cells collected from each cell line is in a 1:1:1 ratio based on the total number of cells used.

For Example: UHT-CMNPs derive by combining 1 part of cell membranes derived from U-251 MG cells, 1 part of cell membranes derived from hCMEC/D3 cells and 1 part of cell membranes derived from T98G cells.

According to a preferred embodiment, the method provides for the combined use of a first and a second cell line, wherein the cell membrane of each cell line is mixed in amounts such that the corresponding number of cells collected from each cell line represents from 0.25 to 4 parts, preferably from 0.5 to 3 parts, preferably from 1 to 2 parts, preferably from 1 to 1.5 parts of the total number of cells used.

Preferably, the cell membrane of each cell line is mixed in amounts such that the corresponding number of cells collected from each cell line is in a 1:1 ratio based on the total number of cells used.

For example: UH-CMNPs derive by combining 1 part of cell membranes derived from U-251 MG cells and 1 part of cell membranes derived from hCMEC/D3 cells.

Preferably, each cell line, before being subjected to the method of the invention, is cultured up to a percentage of confluence ranging from 50% to 80%, more preferably equal to 50%, 55%, 60%, 65%, 70%, 75% and 80% (measured by optical microscopy). The percentage confluence could vary depending on the cell line; the skilled person knows, according to the specificities of the cell line, whether a 50% confluence, a 80% confluence or any % confluence in between is suitable for the aim of the subsequent cell membrane extraction step.

Once the desired confluence is reached, each cell line is trypsinized and preferably collected in numbers ranging from 3.5 × 10⁶ and 100 ×10⁶. The number of cells could vary according to the number of cell lines used/combined; the skilled person would easily determine the cell number needed from each cell line, according to the specific features of each cell line.

Once collected, the supernatant is removed, and the cells are preferably re-dispersed in sterile water inside a plastic vessel (e.g. falcon tube), which is then preserved for at least 1h, preferably from 1h to 4h, at -20°C or -80°C. Each cell line should be preferably stored in a different plastic vessel.

### Step II: lysing the cell culture of each cell line to obtain lysates of each cell line.

For the aim of the invention, cells can be lysed using chemical, biological (for example by lysozyme) or mechanical methods known to the skilled person and available in literature (https://www.thermofisher.com/it/en/home/life-science/protein-biology/protein-biology-learning-center/protein-biology-resource-library/pierce-protein-methods/traditional-methods-cell-lysis.html).

Preferably, the cell culture of each cell line is lysed separately (meaning each cell line in a different vessel).

Preferably, cells are lysed using mechanical methods selected from the group consisting of extrusion, sonication, homogenization, freezing-thawing, electroporation, mechanical disruption and cell lysis by means of additives/facilitators. Cell lysis by means of additives/facilitators can, for example, be promoted by suspending cells in a hypotonic buffer, which cause cells to swell and burst more readily under physical shearing.

Even more preferably, freeze-thawing is employed as the preferred lysing technique. Preferably, step (II) provides for lysing the cell culture of each cell line to obtain lysates of each cell line in sterile distilled water.

According to a preferred embodiment, the lysing step (II) comprises the sub-steps of
- lysing the culture of each cell line from 2 to 4 times, preferably 3 times to obtain preliminary lysates of each cell line; and
- homogenising the preliminary lysate of each cell line at an external air pressure ranging from 0.110 to 0.165 MPa (16 to 24 psi) (homogenising pressure ranging from 33.1 to 49.6 MPa (4,800 to 7,200 psi)), with a number of passages ranging from 6 to 14, to obtain the lysate of each cell line.

According to a preferred embodiment the preliminary lysates of each cell line are suspended in an aqueous medium before homogenisation; said aqueous medium being preferably sterile distilled water. The resulting lysate of each cell line is thus a suspension in sterile distilled water.

Preferably the sterile distilled water used for the purpose of the invention has a pH < 7.5, preferably 5 < pH < 7.5, preferably 5 < pH < 6.5, preferably about 5.5 pH units.

It is noted that the external air pressure is the pressure value set by the technician on the instrument; whilst the homogenising pressure is the actual pressure within the instrument.

Preferably, homogenisation is performed at an external air pressure ranging from 0.124 to 0.152 MPa (18 to 22 psi) (homogenising pressure 37.2-45.5 MPa (5,400-6,600 psi)), preferably from 0.131 to 0.145 MPa (19 to 21 psi) (homogenising pressure 39.3-43.4 MPa (5,700-6,300 psi)), preferably at 0.138 MPa (20 psi) (homogenising pressure 41.4 MPa (6,000 psi)).

Preferably, the number of passages through the homogeniser valve ranges from 8 to 12, preferably from 9 to 11, preferably the number of passages is 10.

It has to be noted that the number of passages and the homogenisation pressure where defined by the Applicant with a homogeniser having a dynamic homogenizing valve. Nonetheless, given the operating conditions defined by the inventors on a homogeniser so designed, the person skilled in the art would have no undue burden finding the suitable operating conditions to be applied to the sample when using a homogeniser with a different design for the same purposes.

Preferably, homogenisation is performed at a temperature ranging from 20 °C to 28 °C, preferably from 20 °C to 26 °C, preferably from 22 °C to 26 °C, preferably at 24 °C.

### Preferably, the homogeniser employed in step II is a high-pressure homogeniser.

The applicants believe that the combination of lysing, preferably performed by freeze-thawing, and homogenisation is particularly advantageous for the aim of the invention, as it allows control of both the size and the successive loading of the nanoparticles.

### Step III: extracting cell membranes from the lysate of each cell line to obtain cell membrane dispersions of each cell line.

Preferably, step (III) provides for the extraction of cell membranes from the lysate of each cell line to obtain cell membrane dispersions of each cell line in sterile distilled water.

Once each cell line lysate is obtained, the lysates are submitted to the extraction step, preferably containing the following sub-steps:
- centrifuging 1 to 4 times the lysate of each cell line at 4,000 to 12,000 rpm for 4 to 12 minutes at 4°C, collecting each time the supernatant;
- centrifuging 2 to 4 times the supernatant at 2,000 to 8,000 rpm for 8 to 12 minutes at 4°C, using a centrifugal filter with a molecular cut off of from 50 kDa to 150 kDa, preferably from 50 kDa to 100 kDa, collecting each time the non-filtered fraction of the supernatant, in order to obtain the cell membrane dispersion of each cell line.

According to a preferred embodiment, cell membranes are extracted from each cell line separately.

According to a preferred embodiment, the non-filtered fraction of the supernatant is each time re-dispersed in an aqueous medium; this procedure simplifies and optimise the filtration procedure of the sample and allows to obtained cell membrane aqueous dispersions of each cell line.

The aqueous medium suitable for this purpose is preferably sterile distilled water. Preferably the sterile distilled water used for the purpose of the invention has a pH < 7.5, preferably 5 < pH < 7.5, preferably 5 < pH < 6.5, preferably about 5.5 pH units.

Preferably, the first centrifugation sub-step is performed at revolutions per minute ranging from 6,000 to 12,000 rpm, preferably from 8,000 to 11,000 rpm, preferably at 10,000 rpm.

Preferably, the first centrifugation sub-step is performed for 6 to 12 minutes, preferably for 8 to 12 minutes, preferably for 9 to 11 minutes, preferably for 10 minutes.

Preferably, the second centrifugation sub-step is performed at revolutions per minute ranging from 3,000 to 7,000, preferably from 4,000 to 6,000, preferably at 5,000.

Preferably, the second centrifugation sub-step is performed for 6 to 12 minutes, preferably for 8 to 12 minutes, preferably for 9 to 11 minutes, preferably for 10 minutes.

The Applicant believes that the use of filtration techniques in combination with the subsequent homogenisation of the mixed dispersion is particularly advantageous for the aim of the invention as it results in a very simplified and scalable procedure.

### Step IV: mixing from 0.5 to 4 parts by weight of the cell membrane dispersion of each cell line, and adding an aqueous solution or dispersion containing a therapeutic or diagnostic ingredient to obtain a mixed dispersion.

According to a preferred embodiment, the aqueous solution or dispersion containing the therapeutic or diagnostic ingredient contains as solvent or dispersant sterile water.

Preferably the sterile distilled water used for the purpose of the invention has a pH < 7.5, preferably 5 < pH < 7.5, preferably 5 < pH < 6.5, preferably about 5.5 pH units.

Preferably, the therapeutic ingredient is selected from the group consisting of: metabolic inhibitors (e.g., 6-Phosphofructo-2-kinase/fructose-2,6-bisphosphatase), oxidative stress inhibitors (e.g., L-N6-(1-iminoethyl)-lysine), antioxidants (e.g. , vitamin A), anti-inflammatory agents (e.g., nimesulide), anticancer agents (erlotinib), neuroprotective agents (e.g., retinoic acid), antibiotics (e.g., amoxicillin), antidepressants (e.g. sertraline), anticoagulants (e.g., warfarin), antifungals (e.g., fluconazole), anti-diabetic agents (e.g., voglibose), anti-hypertensive agents (e.g., losartan).

According to a preferred embodiment the therapeutic ingredient has a molecular mass ranging from 100 to 900 Da, preferably from 200 Da to 500 Da.

Preferably, the diagnostic ingredient is selected from the group consisting of contrast agents, radiopharmaceuticals.

Examples of suitable contrast agents are fludeoxyglucose, fluoromisonidazole, fluorothymidine F-18, gallium citrate Ga-67 and ioxehol.

According to a preferred embodiment the diagnostic ingredient has a molecular mass ranging from 100 to 900 Da, preferably ranging from 700 to 900 Da.

More preferably, the therapeutic or diagnostic ingredient is:
a hydrophobic ingredient having a partition coefficient ranging from 0.5 and 2.5, or
a hydrophilic ingredient having a partition coefficient ranging from -3 And -0.5, and preferably functionalised with state of the art bioconjugation strategies. Preferably, suitable functional groups are selected from the group consisting of: amines, carboxylic acids, thiols and combinations thereof.

### Step V: homogenising the mixed dispersion at a homogenising pressure ranging from 165.5 MPa to 344.7 MPa (24,000 psi to 50,000 psi) (circa 0.552-1.151 MPa (80-167 psi) external air pressure), with a number of passages ranging from 6 to 14, to form a dispersion containing a plurality of nanoparticles.

Once the cell membrane dispersion obtained from each cell line are mixed together, along with a solution or a dispersion of the therapeutic or diagnostic ingredient, a mixed dispersion is obtained.

Such a mixed dispersion is submitted to a homogenisation at a homogenising pressure preferably ranging from 179.3 MPa to 330.9 MPa (26,000 psi to 48,000 psi) (0.600-1.103 MPa (87-160 psi) external pressure), preferably from 193.1 MPa to 303.4 MPa (28,000 psi to 44,000 psi) (0.641-1.014 MPa (93-147 psi) external pressure), from 206.8 MPa to 289.6 MPa (30,000 psi to 42,000 psi) (0.689-0.965 MPa (100-140 psi) external pressure), from 220.6 MPa to 275.8 MPa (32,000 psi to 40,000 psi) (0.738-0.917 MPa (107-133 psi) external pressure), from 234.4 MPa to 262.0 MPa (34,000 psi to 38,000 psi) (0.779-0.876 MPa (113-127 psi) external pressure), preferably from 241.3 MPa to 255.1 MPa (35,000 psi to 37,000 psi) (0.800-0.848 MPa (116-123 psi) external pressure), preferably at 248.2 MPa (36,000 psi) (0.827 MPa (120 psi) external pressure).

Preferably, the number of passages through the homogeniser valve ranges from 8 to 12, preferably from 9 to 11, preferably the number of passages is 10.

Preferably, homogenisation is performed at a temperature ranging from 2 °C to 8 °C, preferably from 2 °C to 6 °C, preferably from 3 °C to 5 °C, preferably at 4 °C.

Preferably, the homogeniser employed in step V is a high-pressure homogeniser.

### Step VI: isolating the plurality of nanoparticles from the dispersion containing them.

Isolation of the plurality of particles from the dispersion obtained from step V can be performed by means of isolation techniques known to the skilled person and available in literature, like for example ultracentrifugation, microfluidics, magnetic beads and filtration-based isolation methods.

According to a preferred embodiment, the isolation step VI is performed by repeating the sub-steps of the extraction step III on the dispersion containing the plurality of nanoparticles.

In other words, the isolation step VI comprises the sub-steps of:
- centrifuging 1 to 4 times the dispersion containing the plurality of nanoparticles at 4,000 to 12,000 rpm for 4 to 12 minutes at 4°C, collecting each time the supernatant;
- centrifuging 2 to 4 times the supernatant at 2,000 to 8,000 rpm for 8 to 12 minutes at 4°C, using a centrifugal filter with a molecular cut off of 50-150 kDa, preferably 50-100 kDa, collecting each time the non-filtered fraction of the supernatant, in order to obtain a purified sample of nanoparticles.

According to a preferred embodiment, the non-filtered fraction of the supernatant is each time re-dispersed in an aqueous medium; this procedure simplifies and optimise the filtration procedure of the sample.

The aqueous medium suitable for this purpose is sterile distilled water. Preferably the distilled water has a pH < 9, preferably 5 < pH < 9, preferably 5 < pH < 7.5, preferably 5 < pH < 6.5, preferably about 5.5 pH units.

Preferably, the first centrifugation sub-step is performed at revolutions per minute ranging from 4,000 to 8,000 rpm, preferably from 4,000 to 6,000 rpm, preferably at 5,000 rpm.

Preferably, the first centrifugation sub-step is performed for 6 to 12 minutes, preferably for 8 to 12 minutes, preferably for 9 to 11 minutes, preferably for 10 minutes.

Preferably, the second centrifugation sub-step is performed at revolutions per minute ranging from 3,000 to 7,000, preferably from 4,000 to 6,000, preferably at 5,000.

Preferably, the second centrifugation sub-step is performed for 6 to 12 minutes, preferably for 8 to 12 minutes, preferably for 9 to 11 minutes, preferably for 10 minutes.

According to a preferred embodiment, the purified sample of nanoparticles obtained from the second centrifugation sub-step of isolation step VI can be additionally filtered with a 0.20 µm sterile syringe filter, to further sterilize and remove the big particles from the sample. The sample obtained from the additional filtration can be subsequently stored at 4°C for further use.

### Nanoparticles

Another object of the present invention are cell-membrane derived nanoparticles comprising
- an outer shell containing cell membranes, each cell membrane deriving from two to four different cell line,
- an inner core made of an aqueous solution or dispersion containing a therapeutic or diagnostic ingredient,

wherein the shell contains from 0,25 to 4 parts of the cell membrane of each cell line,
characterised by having a percentage loading capacity ranging from 10% to 25%.

As previously described, the amount of cell membranes is quantified in number of cells, as the presumption is that the number of source cells for extraction corresponds to the number of extracted membranes. In other words, the amount in parts of cell membranes contained in the shell of the nanoparticles equals the cell number of the corresponding source cell line, based on the total cell number of all the source cell line used.

According to a preferred embodiment, the cell-membrane derived nanoparticles are obtained with the method described previously. In this sense, any information given above with reference to the method applies, mutatis mutandis, to the nanoparticles of the invention.

The cell-membranes nanoparticles obtained with the method of the invention are characterised by a percentage loading capacity ranging from 10% to 25%.

In general, by nanoparticle is meant a nanomaterial with any external dimensions ranging from approximately 1 nm to 100 nm, consisting for 50% or more of particles having a size between 1 nm-100 nm (EU directive 2011/696/EU).

The nanoparticles of the invention have a numerical (number weighted) average size lower than 100 nm, as calculated from high-resolution scanning electron microscopy (SEM).

Preferably, the nanoparticles size calculated from high resolution scanning electron microscopy (SEM) images ranges from 1 nm to 250 nm, preferably from 10 nm to 250 nm, preferably from 20 nm to 250 nm, preferably from 20 nm to 220 nm.

Preferably, the intensity-based average hydrodynamic diameter of the nanoparticles measured in water, as calculated from dynamic light scattering (DLS) measurements ranges from 200 to 250 nm.

Preferably, the surface charge of the nanoparticles, as calculated by DLS measurements in water, ranges from -10 mV to -25mV.

Preferably, the nanoparticle of the invention has a loading capacity ranging from 10% to 25%, preferably from 12% to 25%, preferably from 16 to 25%, preferably from 18% to 25%, preferably from 20% to 25%, preferably from 22% to 25%.

It is noted that loading capacity is the amount of drug loaded per unit weight of the nanoparticle, indicating the percentage of mass of the nanoparticle that is due to the encapsulated drug. Loading capacity (LC%) can be calculated by the amount of total entrapped drug divided by the total nanoparticle weight. In drug delivery, yield, given as a percent, is a reflection of the amount of drug delivered per amount encapsulated. (https://www.sigmaaldrich.com/technical-documents/articles/materials-science/drug-delivery/drug-delivery-questions.html#:∼:text=Loading%20capacity%20is%20the%20amount,by%20the%2 0total%20nanoparticle%20weight)

Preferably, the cell-membrane derived nanoparticles are for medical and/or diagnostic use; particularly, they can be used as drug delivery systems for medical or diagnostic or theranostic (medical and diagnostic) use.

Preferably, whether the nanoparticles are provided for medical use, they can be addressed to the treatment of various diseases; for example, any of the diseases selected from the group consisting of: cancer, brain diseases, ischemic stroke, Parkinson's disease, Alzheimer's disease, diabetes, intervertebral disc degeneration, myocardial infarction, chronic obstructive pulmonary disease.

According to a preferred embodiments, combinations of cell lines selected from the group consisting of U-251 MG, U87 MG, T98G, SH-SY5Y, neuronal-like SH-SY5Y, hCMEC/D3 and combinations thereof are provided for medical use, preferably for the treatment of cancer, ischemic stroke, Parkinson's disease, Alzheimer's disease, diabetes, intervertebral disc degeneration, myocardial infarction or chronic obstructive pulmonary disease.

Preferably, nanoparticles obtained from (U-251 MG + hCMEC/D3) and from (U-251 MG + hCMEC/D3 + T98G) can be used for the treatment of brain diseases including glioblastoma.

Preferably, nanoparticles obtained from hCMEC/D3 + Neural cells (SH-SY5Y differentiated) can be used for the treatment of ischemic stroke.

Preferably, whether provided for diagnostic use, the nanoparticles can be addressed to the diagnosis of various disease, such as thyroid disease, phaeochromocytoma, neuroblastoma, bone metastases, neuroendocrine tumours.

More preferably, the nanoparticles are for use in the treatment and/or diagnosis of cerebral diseases, preferably selected from the group consisting of brain tumours, ischemic stroke, Parkinson's disease and Alzheimer's disease.

According to a preferred embodiment, the nanoparticles are for use in the treatment and/or diagnosis of brain tumours selected from the group consisting of gliomas and neuroblastomas.

### EXAMPLE

For illustrative and non-limiting purposes, it is presented herewith an example of the method and of the nanoparticles according to the invention.

### 1. Fabrication protocol of Fused-Cell Membranes NanoParticles (F-CMNPs), obtained from 3 different cell lines, loaded with the glucose inhibitor (2E)-3-(3-Pyridinyl)-1-(4-pyridinyl)-2-propen-1-one (3PO), used as a model drug.

1. Culture hCMEC/D3, U251, and SY-SY5Y cells until 80%, 80% and 50% of confluence, respectively
2. Trypsinize each cell line separately and collect the cells (10x106 for each cell line) by centrifugation at 2000 rpm for 5 min at 25 °C.
3. Remove the supernatants, and re-disperse the cells in 1 ml of sterile distilled water inside a plastic vessel (e.g., falcon tube), which subsequently place at -20 °C or -80 °C for at least 1h. Each cell line should be in a different falcon tube.
4. Perform a freeze-thaw cycle for 3 times for each falcon tube.
5. Add 1 ml more of sterile distilled water and transfer the cells in a high-pressure homogenizer. Pass the lysed cells 10 times at 0.138 MPa (20 psi) external air pressure (41.4 MPa (6000 psi) homogenizing pressure). Each cell line separately.
6. Spin down the lysed cells at 10000 rpm for 10 min, and collect the supernatant. Repeat the spinning 2 more times, and always collect the supernatant. Each cell line should be cleaned separately.
7. Subsequently transfer the samples in Amicon filters with a molecular weight cut-off equal to 100 kDa, and spin down 3 times at 5000 rpm for 10 min. Always re-disperse in sterile distilled water. At the end of the third spinning, re-disperse each of the material on the upper part of the Amicon filters in 333 µl of sterile distilled water.
8. Mix the three extracted cell membranes (ratio 1:1:1) and add the drug [(2E)-3-(3-Pyridinyl)-1-(4-pyridinyl)-2-propen-1-one)] dispersed in 1 ml of sterile distilled water. For unloaded nanoparticles just mix the cell membranes at a ratio 1:1:1.
9. Transfer the mixed dispersion in the high-pressure homogenizer and homogenize the (mixed) sample at 0.827 MPa (120 psi) (248.2 MPa (36000 psi) homogenizing pressure) keeping the number of passages (10 passages) the same. (The homogenizing pressure, as described by the manufacturer, is 300 times higher than the external air pressure).
10. Spin down 2 times at 5000 rpm for 10 min, and collect the supernatant.
11. Transfer the samples in Amicon filters with a molecular weight cut-off equal to 100 kDa and spin down 3 times at 5000 rpm. This time keep the precipitate in the upper part of the filter, and re-disperse in 500 µl sterile distilled water.
12. Filter the sample using a syringe filter of 0.2 µm, and store at 4 °C until further use. The solution at the bottom of the Amicon filters can be used for the quantification of the loading.

### 2. Nanoparticle prototype - characterisation

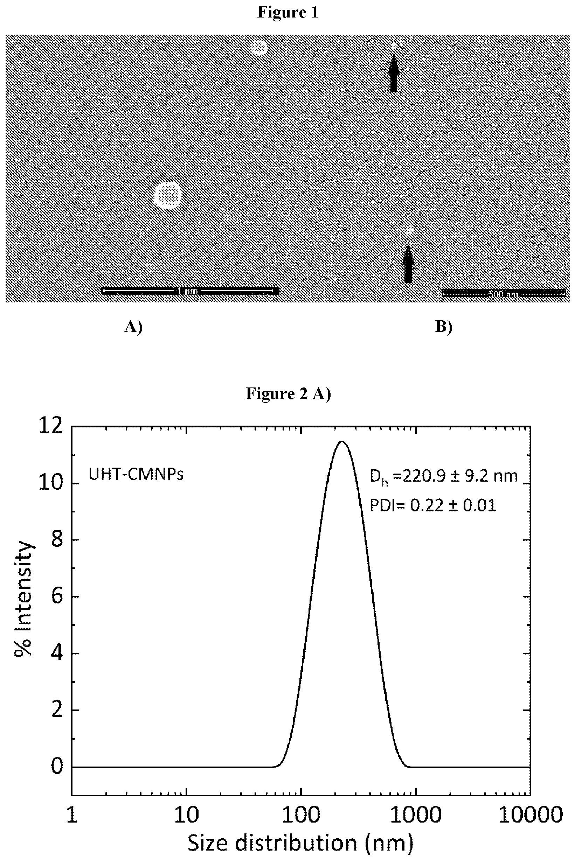

The fabricated F-CMNPs have a spherical morphology (Figure 1) and an average size < 200 nm as it was calculated from high resolution scanning electron microscopy (SEM) images.

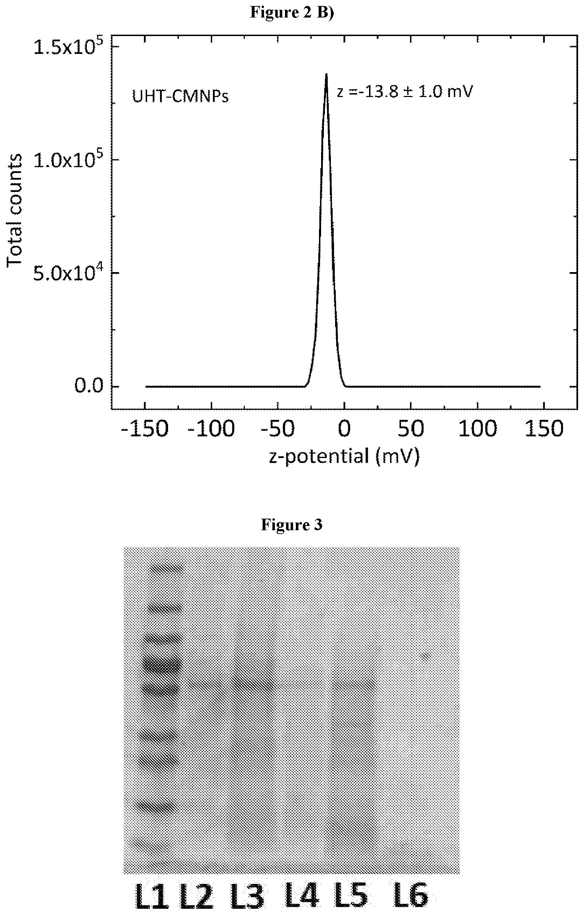

The average hydrodynamic diameter of the CMNPs, as it was calculated from dynamic light scattering (DLS) measurements (Figure 2), ranges from 200 to 220 nm, while their surface charge ranges from -10 to -15 mV.

The presence of proteins on the surface of the F-CMNPs was demonstrated using sodium dodecyl sulphate poly acrylamide gel electrophoresis assay (SDS-PAGE) and the results are presented in Figure 3. As a positive control we used lipid-based nanoparticles made from sphingomyelin and functionalized with the angiopep-2 peptide (SM-AP2).

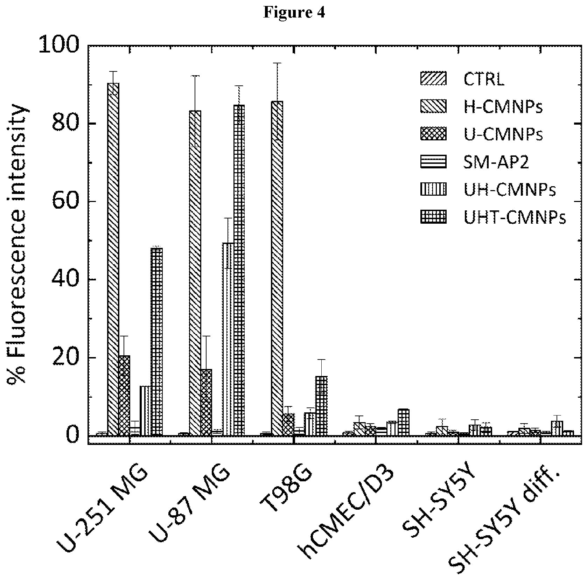

The multi-targeting ability of the F-CMNPs was demonstrated after performing internalization experiments, using different cell lines as depicted in Figure 4. The internalization data suggest that the homologous targeting ability can be used only in specific cell lines and cannot be generalized for the majority of them. In addition, these data suggest that a proper combination of cell membranes and subsequently the proper combination of surface receptors can result to a faster and more specific internalization to several cell lines.

From these preliminary data it can also be assumed that the proper combination of surface proteins not only provides a multi-targeting ability, but can also provide a multi-evasion ability from the cells of the phagocytic systems.

Finally, the proper combination of cell membranes and of the subsequent surface receptors can enhance the internalization by specific cell lines, especially in comparison to the robust peptide-targeting technique using specific peptides like angiopep-2.

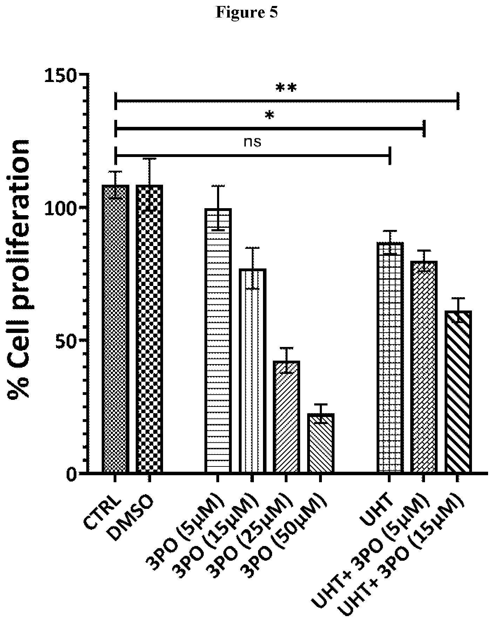

The ability of the F-CMNPs to act as drug delivery systems was also studied. The results that are presented in Figure 5, showed that the F-CMNPs are highly biocompatible, but can also kill cancer cells if the proper chemotherapeutic is encapsulated. As a proof of concept, we loaded the glucose inhibitor (2E)-3-(3-Pyridinyl)-1-(4-pyridinyl)-2-propen-1-one (3PO) in the F-CMNPs. The data in Figure 5 show that at a concentration of the inhibitor equal to 5 µM, the free inhibitor is not affecting the U-251 cells' proliferation, in contrast to the encapsulated one (at the same concentration as the free inhibitor as calculated by high pressure liquid chromatography), that reduces the cells' proliferation by 60% after 72 h.

All the presented data show that nanoparticles derived from fused cell membranes can act as an unrivalled versatile theranostic.

### REFERENCES

1. Gong, F., et al., Tumor microenvironment-responsive intelligent nanoplatforms for cancer theranostics. Nano Today, 2020. 32.
2. Tapeinos, C., et al., Advanced Functional Materials and Cell-Based Therapies for the Treatment of Ischemic Stroke and Postischemic Stroke Effects. Advanced Functional Materials, 2019. 30(1).
3. Tang, W., et al., Emerging blood-brain-barrier-crossing nanotechnology for brain cancer theranostics. Chem Soc Rev, 2019. 48(11): p. 2967-3014.
4. M, S.M., et al., External stimulus responsive inorganic nanomaterials for cancer theranostics. Adv Drug Deliv Rev, 2019. 138: p. 18-40.
5. Ilinskaya, A.N. and M.A. Dobrovolskaia, Nanoparticles and the blood coagulation system. Part II: safety concerns. Nanomedicine (Lond), 2013. 8(6): p. 969-81.
6. G., S., et al., Nanocarriers as Potential Targeted Drug Delivery for Cancer Therapy. Nanoscience in Medicine, ed. D. H.;, et al. Vol. 1. 2020: Springer.
7. Blanco, E., H. Shen, and M. Ferrari, Principles of nanoparticle design for overcoming biological barriers to drug delivery. Nat Biotechnol, 2015. 33(9): p. 941-51.
8. Wang, H., et al., Cell membrane biomimetic nanoparticles for inflammation and cancer targeting in drug delivery. Biomater Sci, 2020. 8(2): p. 552-568.
9. Liu, Y., et al., Cell Membrane Coating Technology: A Promising Strategy for Biomedical Applications. Nano-Micro Letters, 2019. 11(1).
10. Tapeinos, C., et al., Cell Membrane-Coated Magnetic Nanocubes with a Homotypic Targeting Ability Increase Intracellular Temperature due to ROS Scavenging and Act as a Versatile Theranostic System for Glioblastoma Multiforme. Adv Healthc Mater, 2019. 8(18): p. e1900612.
11. Chen, M., M. Chen, and J. He, Cancer cell membrane cloaking nanoparticles for targeted co-delivery of doxorubicin and PD-L1 siRNA. Artif Cells Nanomed Biotechnol, 2019. 47(1): p. 1635-1641.
12. Rao, L., et al., Cancer Cell Membrane-Coated Nanoparticles for Personalized Therapy in Patient-Derived Xenograft Models. Advanced Functional Materials, 2019. 29(51).
13. Dong, X., et al., Neutrophil Membrane-Derived Nanovesicles Alleviate Inflammation To Protect Mouse Brain Injury from Ischemic Stroke. ACS Nano, 2019. 13(2): p. 1272-1283.
14. Shi, Y., et al., TRAIL-expressing cell membrane nanovesicles as an anti-inflammatory platform for rheumatoid arthritis therapy. J Control Release, 2020. 320: p. 304-313.

## Claims

1. Method for the production of cell-membrane derived nanoparticles comprising the steps of:
I) providing from two to four cell cultures, each cell culture being of a different cell line, and collecting a predetermined number of cells from each cell line;
II) lysing the predetermined number of cells of each cell line to obtain lysates of each cell line;
III) extracting cell membranes from the lysate of each cell line to obtain cell membrane dispersions of each cell line;
IV) mixing the cell membrane dispersion of each cell line in amounts so that the corresponding predetermined number of cells collected from each cell line represents from 0.25 to 4 parts of the total number of cells collected in step (I), and adding an aqueous solution or dispersion containing a therapeutic or diagnostic ingredient to obtain a mix dispersion;
V) homogenising the mix dispersion at an external air pressure ranging from 0.552 to 1.151 MPa (80 to 167 psi), with a number of passages ranging from 6 to 14, to form a dispersion containing a plurality of nanoparticles;
VI) isolating the plurality of nanoparticles from the dispersion containing them.

2. Method according to claim 1, wherein the lysing step II comprises the sub-steps of
- lysing the culture of each cell line from 2 to 4 times to obtain preliminary lysates of each cell line;
- homogenising the preliminary lysate of each cell line at a line at an external air pressure ranging from 0.110 to 0.165 MPa (16 to 24 psi), with a number of passages ranging from 6 to 14 to obtain the lysate of each cell line.

3. Method according to claim 1 or 2, wherein lysing is performed by a mechanical method selected from the group consisting of: extrusion, sonication, homogenization, freezing-thawing, electroporation, mechanical disruption and cell lysis by means of additives/facilitators.

4. Method according to any one of claims from 1 to 3, wherein the extraction step III comprises the sub-steps of
- centrifuging 1 to 4 times the lysate of each cell line at 4,000 to 12,000 rpm for 4 to 12 minutes at 4°C, collecting each time the supernatant;
- centrifuging 2 to 4 times the supernatant at 2,000 to 8,000 rpm for 8 to 12 minutes at 4°C, using a centrifugal filter with a molecular cut off of 100 kDa, collecting each time the non-filtered fraction of the supernatant, in order to obtain the cell membrane dispersion of each cell line.

5. Method according to claim 4, wherein the isolation step VI is performed by repeating the sub-steps of the extraction step III on the dispersion containing the plurality of nanoparticles, in order to isolate the plurality of nanoparticles from the dispersion.

6. Method according to any one of claims from 1 to 5, wherein each cell line is an animal or human cell line independently selected from the group consisting of primary cell lines and immortalised cell lines.

7. Method according to any one of claims from 1 to 6, wherein the therapeutic ingredient is selected from the group consisting of metabolic inhibitors, oxidative stress inhibitors, antioxidants, anti-inflammatory agents, anticancer agents, neuroprotective agents, antibiotics, antidepressants, anticoagulants, antifungals, anti-diabetic agents, anti-hypertensive agents.

8. Method according to any one of claims from 1 to 6, wherein the diagnostic ingredient is selected from the group consisting of contrast agents, radiopharmaceuticals.

9. Method according to any one of claims from 1 to 8, wherein the therapeutic or diagnostic ingredient has a molecular mass ranging from 100 to 900 Da.

10. Cell-membrane nanoparticles comprising
- an outer shell containing cell membranes deriving from a predetermined cell number of two to four different cell lines, the shell containing cell membranes of each cell line in amounts so that the corresponding predetermined number of cells of each cell line represents from 0.25 to 4 parts of the total cells number of all the cell lines,
- an inner core made of an aqueous solution or dispersion containing a therapeutic or diagnostic ingredient,
**characterised by** having a percentage loading capacity ranging from 10% to 25%.

11. Cell-membrane nanoparticles according to claim 10, obtained with the method according to any one of claims from 1 to 9.

12. Cell-membrane nanoparticles according to claim 10 or 11 for medical and/or diagnostic use.

## Patentansprüche

1. Verfahren zur Herstellung von aus Zellmembranen abgeleiteten Nanopartikeln, umfassend die folgenden Schritte:
I) Bereitstellen von zwei bis vier Zellkulturen, wobei jede Zellkultur aus einer verschiedenen Zelllinie stammt, und Sammeln einer vorgegebenen Anzahl von Zellen aus j eder Zelllinie;
II) Lysieren der vorgegebenen Anzahl von Zellen jeder Zelllinie, um Lysate jeder Zelllinie zu erhalten;
III) Extrahieren Zellmembranen aus dem Lysat jeder Zelllinie, um Zellmembran-Dispersionen jeder Zelllinie zu erhalten;
IV) Mischen der Zellmembran-Dispersion jeder Zelllinie in solchen Mengen, so dass die entsprechende vorgegebene Anzahl von Zellen, die von jeder Zelllinie gesammelt wurden, 0,25 bis 4 Teile der Gesamtzahl der in Schritt (I) gesammelten Zellen darstellt, und Hinzufügen einer wässrigen Lösung oder Dispersion, die einen therapeutischen oder diagnostischen Inhaltsstoff enthält, um eine Mischdispersion zu erhalten;
V) Homogenisieren der Mischdispersion bei einem Aussenluftdruck im Bereich von 0,552 bis 1,151 MPa (80 bis 167 psi), mit einer Anzahl von Wechseln im Bereich von 6 bis 14, um eine Dispersion zu bilden, die eine Mehrzahl von Nanopartikeln enthält;
VI) Isolieren der Mehrzahl von Nanopartikeln aus der sie enthaltenden Dispersion.

2. Verfahren nach Anspruch 1, wobei der Lysierenschritt II die folgenden Unterschritte umfasst
- Lysieren der Kultur jeder Zelllinie 2 bis 4 Mal, um Vorlysate jeder Zelllinie zu erhalten;
- Homogenisieren des Vorlysats jeder Zelllinie in einer Linie bei einem Aussenluftdruck von 0,110 bis 0,165 MPa (16 bis 24 psi), mit einer Anzahl von Wechseln von 6 bis 14, um das Lysat jeder Zelllinie zu erhalten.

3. Verfahren nach Anspruch 1 oder 2, wobei das Lysieren durch ein mechanisches Verfahren durchgeführt wird, das aus der Gruppe ausgewählt ist, die aus: Extrusion, Ultraschall, Homogenisierung, Gefrieren-Auftauen, Elektroporation, mechanischem Aufbrechen und Zelllysieren mittels Additiven/Facilitatoren besteht.

4. Verfahren nach einem der Ansprüche 1 bis 3, wobei der Extraktionsschritt III die folgenden Unterschritte umfasst
- Zentrifugieren 1 bis 4 Mal des Lysats jeder Zelllinie bei 4.000 bis 12.000 U/min für 4 bis 12 Minuten bei 4°C, wobei jedes Mal der Überstand gesammelt wird;
- Zentrifugieren 2 bis 4 Mal des Überstands bei 2.000 bis 8.000 U/min für 8 bis 12 Minuten bei 4°C unter Verwendung eines Zentrifugalfilters mit einem molekularen Cut-Off von 100 kDa, wobei jedes Mal die ungefilterte Fraktion des Überstands gesammelt wird, um die Zellmembran-Dispersion jeder Zelllinie zu erhalten.

5. Verfahren nach Anspruch 4, wobei der Isolierungsschritt VI durch Wiederholen der Unterschritte des Extraktionsschritts III an der Dispersion umfassend die Mehrzahl von Nanopartikeln durchgeführt wird, um die Mehrzahl von Nanopartikeln aus der Dispersion zu isolieren.

6. Verfahren nach einem der Ansprüche 1 bis 5, wobei jede Zelllinie eine tierische oder menschliche Zelllinie ist, die unabhängig aus der Gruppe ausgewählt wird, die aus primären Zelllinien und immortalisierten Zelllinien besteht.

7. Verfahren nach einem der Ansprüche 1 bis 6, wobei der therapeutische Inhaltsstoff aus der Gruppe ausgewählt ist, die aus Stoffwechselinhibitoren, Inhibitoren von oxidativem Stress, Antioxidantien, entzündungshemmenden Mitteln, Antikrebsmitteln, neuroprotektiven Mitteln, Antibiotika, Antidepressiva, Antikoagulantien, Antimykotika, Antidiabetika und Antihypertonika besteht.

8. Verfahren nach einem der Ansprüche 1 bis 6, wobei der diagnostische Hilfsstoff aus der Gruppe der Kontrastmittel und Radiopharmaka ausgewählt ist.

9. Verfahren nach einem der Ansprüche 1 bis 8, wobei der therapeutische oder diagnostische Inhaltsstoff eine Molekülmasse im Bereich von 100 bis 900 Da aufweist.

10. Zellmembran-Nanopartikel umfassend
- eine äußere Hülle, die Zellmembranen enthält, die von einer vorgegebenen Zellanzahl von zwei bis vier verschiedenen Zelllinien ableiten, wobei die Hülle Zellmembranen jeder Zelllinie in solchen Mengen enthält, dass die entsprechende vorgegebene Anzahl von Zellen jeder Zelllinie 0,25 bis 4 Teile der Gesamtzellanzahl aller Zelllinien darstellt,
- einen inneren Kern aus einer wässrigen Lösung oder Dispersion, die einen therapeutischen oder diagnostischen Inhaltsstoff enthält,
**gekennzeichnet durch** eine Prozent-Ladekapazität von 10 % bis 25 %.

11. Zellmembran-Nanopartikel nach Anspruch 10, erhalten mittels dem Verfahren nach einem der Ansprüche 1 bis 9.

12. Zellmembran-Nanopartikel nach Anspruch 10 oder 11 zur medizinischen und/oder diagnostischen Verwendung.

## Revendications

1. Procédé de production de nanoparticules dérivées de membrane cellulaire comprenant les étapes suivantes :
I) fournir de deux à quatre cultures cellulaires, chaque culture cellulaire étant d'une lignée cellulaire différente, et collecter un nombre prédéterminé de cellules de chaque lignée cellulaire ;
II) lyser le nombre prédéterminé de cellules de chaque lignée cellulaire pour obtenir des lysats de chaque lignée cellulaire ;
III) extraire les membranes cellulaires du lysat de chaque lignée cellulaire pour obtenir des dispersions de membranes cellulaires de chaque lignée cellulaire ;
IV) mélanger la dispersion de membrane cellulaire de chaque lignée cellulaire en quantités telles que le nombre prédéterminé correspondant de cellules prélevées sur chaque lignée cellulaire représente de 0,25 à 4 parties du nombre total de cellules prélevées à l'étape (I), et ajouter une solution ou une dispersion aqueuse contenant un ingrédient thérapeutique ou diagnostique afin d'obtenir une dispersion de mélange ;
V) homogénéiser la dispersion de mélange au niveau d'une pression d'air externe comprise entre 0,552 et 1,151 MPa (80 à 167 psi), avec un nombre de passages compris entre 6 et 14, pour former une dispersion contenant une pluralité de nanoparticules ;
VI) isoler la pluralité de nanoparticules de la dispersion qui les contient.

2. Procédé selon la revendication 1, dans lequel l'étape de lyse II comprend les sous-étapes suivantes
- lyser la culture de chaque lignée cellulaire de 2 à 4 fois pour obtenir des lysats préliminaires de chaque lignée cellulaire ;
- homogénéiser le lysat préliminaire de chaque lignée cellulaire sur une lignée à une pression d'air externe comprise entre 0,110 et 0,165 MPa (16 à 24 psi), avec un nombre de passages compris entre 6 et 14 pour obtenir le lysat de chaque lignée cellulaire.

3. Procédé selon la revendication 1 ou 2, dans lequel la lyse est effectuée par une méthode mécanique choisie dans le groupe constitué par : l'extrusion, la sonication, l'homogénéisation, la congélation-décongélation, l'électroporation, la perturbation mécanique et la lyse cellulaire au moyen d'additifs/de facilitateurs.

4. Procédé selon l'une quelconque des revendications 1 à 3, dans lequel l'étape d'extraction III comprend les sous-étapes suivantes
- centrifuger de 1 à 4 fois le lysat de chaque lignée cellulaire à 4 000 à 12 000 rpm pendant 4 à 12 minutes à 4°C, en collectant à chaque fois le surnageant ;
- centrifuger de 2 à 4 fois le surnageant à 2 000 à 8 000 rpm pendant 8 à 12 minutes à 4°C, à l'aide d'un filtre centrifuge avec une coupure moléculaire de 100 kDa, en collectant à chaque fois la fraction non filtrée du surnageant, afin d'obtenir la dispersion de membrane cellulaire de chaque lignée cellulaire.

5. Procédé selon la revendication 4, dans lequel l'étape d'isolation VI est réalisée en répétant les sous-étapes de l'étape d'extraction III sur la dispersion contenant la pluralité de nanoparticules, afin d'isoler la pluralité de nanoparticules de la dispersion.

6. Procédé selon l'une quelconque des revendications 1 à 5, dans lequel chaque lignée cellulaire est une lignée cellulaire animale ou humaine choisie indépendamment dans le groupe constitué par les lignées cellulaires primaires et les lignées cellulaires immortalisées.

7. Procédé selon l'une quelconque des revendications 1 à 6, dans lequel l'ingrédient thérapeutique est choisi dans le groupe constitué par les inhibiteurs métaboliques, les inhibiteurs du stress oxydatif, les antioxydants, les agents anti-inflammatoires, les agents anticancéreux, les agents neuroprotecteurs, les antibiotiques, les antidépresseurs, les anticoagulants, les antifongiques, les agents antidiabétiques, les agents antihypertenseurs.

8. Procédé selon l'une quelconque des revendications 1 à 6, dans lequel l'ingrédient diagnostique est choisi dans le groupe constitué par les agents de contraste, les produits radiopharmaceutiques.

9. Procédé selon l'une quelconque des revendications 1 à 8, dans lequel l'ingrédient thérapeutique ou diagnostique a une masse moléculaire comprise entre 100 et 900 Da.

10. Nanoparticules de membrane cellulaire comprenant
- une enveloppe extérieure contenant des membranes cellulaires provenant d'un nombre prédéterminé de cellules de deux à quatre lignées cellulaires différentes, l'enveloppe contenant des membranes cellulaires de chaque lignée cellulaire en quantités telles que le nombre prédéterminé correspondant de cellules de chaque lignée cellulaire représente de 0,25 à 4 parties du nombre total de cellules de toutes les lignées cellulaires,
- un noyau interne constitué d'une solution ou d'une dispersion aqueuse contenant un ingrédient thérapeutique ou diagnostique,
**caractérisées par** une capacité de charge en pourcentage comprise entre 10 et 25 %.

11. Nanoparticules de membrane cellulaire selon la revendication 10, obtenues par le procédé selon l'une quelconque des revendications de 1 à 9.

12. Nanoparticules de membrane cellulaire selon la revendication 10 ou 11 pour une utilisation médicale et/ou diagnostique.
